# EUROPÄISCHE PATENTSCHRIFT

(11) **EP 0 463 620 B1**
(45) Veröffentlichungstag und Bekanntmachung des Hinweises auf die Patenterteilung: **09.08.1995**
(21) Anmeldenummer: 91110614.4
(22) Anmeldetag: 26.06.1991
(51) Int. Cl.: A61B 5/08, A61B 5/0205, A61B 5/113

(54) **Sauerstoff-Puls-Respirograf**
Recorder for pulse and oxygen in breathing
Enregistrement pour le pouls et la teneur en oxygène dans la respiration

(30) Priorität: 29.06.1990 DE 4020823
(43) Veröffentlichungstag der Anmeldung: 02.01.1992
(73) Patentinhaber: Ryba, Jan, D-67063 Ludwigshafen (DE)
(72) Erfinder: Ryba, Jan, D-67063 Ludwigshafen (DE)
(74) Vertreter: Patentanwälte Möll und Bitterich

(56) Entgegenhaltungen:
- EP-A- 0 371 424
- FR-A- 2 575 917
- GB-A- 2 225 459
- US-A- 4 860 759

## Beschreibung

Die Erfindung betrifft einen Sauerstoff-Puls-Respirografen für die ambulante aber auch stationäre Anwendung, enthaltend eine Langzeit-Aufzeichnungseinrichtung.

Monitoren für die Überwachung der Sauerstoffsättigung des Blutes, des Pulses, des Herzmuskels, der Atemtätigkeit usw. sind bekannt. Respirografen oder Atemmonitoren werden eingesetzt zur Patientenüberwachung, insbesondere auf Intensivstationen, aber auch in sogenannten Schlaflabors. Sie dienen dazu, die Atemfrequenz des Patienten zu messen, die Atemkurve aufzuzeichnen und Unregelmäßigkeiten der Atmung anzuzeigen, insbesondere den Atemstillstand (Apnoe).

Aufgrund ihrer Einsatzbestimmung waren die Geräte zunächst als Standgeräte konzipiert. Die Messung der Atemtätigkeit erfolgt entweder elektrisch über die Impedanzänderungen im Thoraxbereich oder mit Hilfe eines Brustgurtes, der mit einem entsprechenden elektromechanischen Dehnungssensor ausgerüstet ist. Ein derartiges Gerät ist beispielsweise beschrieben in der DE-OS 24 18 910.

Um weitere, für die ärztliche Behandlung wichtige Meßwerte zu gewinnen, sind die bekannten Respirografen mit weiteren Meßmitteln ausgerüstet. Meist handelt es sich um Elektroden zur Aufnahme eines Elektrokardiogramms (EKG). Ein solches Gerät ist beispielsweise beschrieben in der DE-OS 24 18 910.

Seit bekannt ist, daß Schnarcher besonders von Apnoe bedroht sind, sind Überwachungsgeräte entwickelt worden, die mit einem Mikrophon, z. B. einem Kehlkopfmikrophon ausgerüstet sind. Diese reagieren auf das Ausbleiben der regelmäßigen Schnarchgeräusche, z. B. im Falle eines Oropharynxverschlusses. Ein derartiges Gerät, welches neben einem Kehlkopfmikrophon auch EKG-Elektroden besitzt und in einem tragbaren Gehäuse mit einer Langzeit-Aufzeichnungseinrichtung untergebracht ist, ist beschrieben in der EP-A-0 371 424. Dieses Gerät ist auch handelsüblich. Es eignet sich aber nicht als Respirograf. Seine Einsatzdauer beträgt theoretisch 18 Stunden; in der Praxis werden nur 12 Stunden erreicht.

Patienten, bei denen der Arzt aufgrund der beobachteten Symptome eine Apnoe-Gefährdung vermutet, werden in besondere Schlaflabors eingewiesen, wo eine Überwachung der Atemtätigkeit und weiterer Körperfunktionen während des Schlafens stattfinden kann. Die Zahl der Schlaflabors und damit die Zahl der zu untersuchenden Patienten ist beschränkt; außerdem ist eine solche Untersuchung sehr kostspielig und für den Patienten aufwendig. Auch ist bekannt, daß Apnoen nicht nur nachts und im Schlaf auftreten, sondern auch tagsüber; diese werden in Schlaflabors ebensowenig erfaßt wie mit ambulanten Kurzzeit-Aufzeichnungsgeräten.

Der vorliegenden Erfindung liegt die Aufgabe zugrunde, einen Sauerstoff-Puls-Respirografen anzugeben, der es gestattet, die Atemtätigkeit, die Sauerstoffsättigung des Blutes und den Puls sowie bei Bedarf eine Reihe weiterer, für die ärztliche Diagnostik wichtiger Körperfunktionen des Patienten rund um die Uhr zu erfassen, wobei der Patient seinen normalen Tagesablauf beibehalten kann.

Diese Aufgabe wird gelöst durch einen Sauerstoff-Puls-Respirografen mit den Merkmalen gemäß Anspruch 1.

Es handelt sich erfindungsgemäß um ein Gerät, dessen Stromquelle und Aufzeichnungseinrichtung für einen 24- bis 48-stündigen Dauerbetrieb ausgelegt sind, analog dem 24-h-Langzeit-EKG. Die Sensoren und Elektroden werden vom Arzt oder vom ärztlichen Hilfspersonal in der Praxis angebracht und auf einwandfreie Funktion kontrolliert. Der Patient selbst kann dann die Praxis verlassen und seiner normalen Tätigkeit nachgehen, ohne durch das tragbare Gerät oder die Sensoren und Elektroden merkbar behindert zu werden.

Dabei prüft der vorhandene Atemfühler, vorzugsweise ein Thermofühler, ob aus Mund und/oder Nase auch wirklich ein Atemhauch austritt oder ob die Atmung unterbrochen ist.

Die Existenz des Pulssensors erlaubt die Überwachung der Herztätigkeit in einem für die Atemüberwachung ausreichenden Maß. Einfache, leichte und betriebssichere Pulssensoren sind handelsüblich. Die Aufnahme eines vollständigen EKG ist im allgemeinen nicht erforderlich. Gegebenenfalls kann das erfindungsgemäße Gerät jedoch auch zusätzlich mit EKG-Elektroden und einem dazugehörigen Meßkanal ausgestattet werden.

Die Verwendung des Blutsauerstoff-Sättigungssensors erlaubt es, die Fälle sicher zu erkennen, bei denen zwar die Atemtätigkeit nicht unterbrochen ist, die Atmung jedoch so flach wird, daß das Blut zu wenig Sauerstoff bekommt, z. B. beim Hypoventilations-Syndrom. Derartige Fälle können mit den bekannten Geräten, die nur mit Brustgurt und/oder EKG-Elektroden ausgerüstet sind, nicht erkannt werden.

Dank des Einsatzes von Brust- und/oder Bauchgurt können sowohl Brustatmung als auch Bauchatmung erfaßt werden. Eine typische Erscheinung bei Apnoen ist, daß Brustkorb und/oder Bauch weiterhin ihre periodischen Bewegungen ausführen können, wobei jedoch infolge eines Oropharynxverschlusses durch Mund und Nase kein Atemhauch strömt. Dies wird vom Atemfühler erkannt. Auch das sogenannte Müller-Manöver wird dank Brust- und Bauchgurt sicher diagnostiziert.

Das Vorhandensein eines pH-Wert-Sensors ermöglicht die Aufnahme von Messwerten aus Speiseröhre und/oder Magen.

Zur Messung von Puls und Blutsauerstoff-Sättigung kann vorzugsweise ein handelsüblicher Kombinationssensor, der unblutig auf der Basis der Lichttransmissionsmessung arbeitet, eingesetzt werden.

In vielen Fällen geht mit der Apnoe eine außergewöhnliche Muskelaktivität einher. Um diese Fälle erfassen zu können, sind gemäß einer Weiterbildung der Erfindung Elektroden zur Aufnahme eines Elektromyogramms vorgesehen.

Um die in anderen Fällen mit einer Apnoe einhergehenden Veränderungen des Drucks in der Speiseröhre erfassen zu können, sind besondere Sensoren vorzusehen, die die Meßwerte aus dem Körper des Patienten aufnehmen und an das Gerät bzw. an dessen Elektronikschaltung weiterleiten.

Schließlich lassen sich mit Hilfe von EEG-Elektroden die unterschiedlichen Wach- und Schlafphasen erfassen und aufzeichnen.

Als Langzeit-Signalspeicher eignen sich Magnetbänder oder Magnetplatten, besonders aber Halbleiterspeicher, z. B. die neuen Mega-Chips, deren Daten mittels Akku gepuffert werden, oder die sogenannten Flash-Cards.

Im Fall einer digitalen Speicherung der gemessenen Werte sind Analog-Digital-Wandler als Anpassungsschaltung zwischen den Sensoren und der Elektronikeinheit erforderlich. Eine programmierbare Zeiteinheit mit akustischem Signal erlaubt die genaue Messung der im Bett verbrachten Zeit ("Total time in bed").

Da eine individuelle Auswertung der Daten, insbesondere in der höchsten Ausbaustufe des Gerätes, durch den Arzt fast unmöglich, auf jeden Fall sehr zeitaufwendig ist, wird diese Aufgabe einem Computer übertragen. Zu diesem Zweck wird das erfindungsgemäße Gerät mit einer geeigneten Datenschnittstelle ausgerüstet. Das Gerät ist somit auch für den stationären Bereich z. B. in einem Krankenhaus geeignet und einsatzfähig.

Der Atemfühler ist je nach Bedarf zusätzlich oder alternativ auch durch ein Volumen-Meßgerät oder in Verbindung mit einem n-CPAP-Gerät durch einen Drucksensor zu ergänzen.

Um das Gerät je nach Bedarf konfigurieren zu können, empfiehlt sich ein modulartiger Aufbau desselben, auch der Elektronikeinheit.

Anhand der schematischen Zeichnung soll die Erfindung näher erläutert werden. Dargestellt ist ein Blockschaltbild eines Sauerstoff-Puls-Respirografen mit Zusatzfunktionen.

In einem tragbaren, möglichst leichten und kleinen Gehäuse 1 sind eine Stromquelle 2, ein Langzeit-Signalspeicher 3, beispielsweise ein akkugepufferter Halbleiterspeicher oder eine sogenannte Flash-Card, und eine Elektronikeinheit 4 untergebracht. Die Elektronikeinheit 4 ist mit einer der Zahl der anschließbaren Sensoren entsprechenden Zahl von Schnittstellen bzw. Anpassungsschaltungen 5 ausgerüstet. Die gespeicherten Daten können über eine Datenschnittstelle 6 an ein Sichtgerät oder einen Auswertungscomputer übertragen werden. Alle Komponenten sind derart aufeinander abgestimmt, daß eine lückenlose Überwachung und Meßwertspeicherung über beispielsweise 24 oder 48 Stunden möglich ist.

Außerhalb des tragbaren Gerätes 1 und mit der Elektronikeinheit 4, 5 üblicherweise über Leitungen verbunden erkennt man eine Anzahl von Sensoren. Im einzelnen handelt es sich um einen Dehnungssensor 11 an einem Brustgurt 10, einen Atemfühler 20, beispielsweise einen Thermosensor oder eine Windfahne, die/der auf den aus Mund und/oder Nase austretenden Atemhauch anspricht, einen weiteren Dehnungssensor 31 an einem Bauchgurt 30, einen Pulssensor 40, einen Blutsauerstoff-Sättigungssensor 41, die bevorzugt in einem Kombinationssensor kombiniert sind und unblutig auf der Basis der Lichttransmissionsmessung arbeiten, Elektroden 50 für die Aufnahme eines Elektromyogramms, einen pH-Sensor 60 zur Aufnahme von Meßwerten aus Speiseröhre und/oder Magen und einen Drucksensor 70 zur Aufnahme von Meßwerten aus der Speiseröhre.

In einer Basisversion mit Thermosensor 20, Pulssensor 40, zusätzlichem Sauerstoffsensor 41, Brust- und/oder Bauchgurt 10, 30 mit Dehnungssensor 11, 31 und Speiseröhrensonde 60 ist das Gerät ein tragbarer, über 24 bis 48 Stunden ambulant und stationär einsetzbarer Puls-Respirograf, mit dem zahlreiche Ursachen der Lungen- und Atmungsprobleme erkannt werden können. Wird der Pulssensor 40 durch EKG-Elektroden 80 ersetzt oder ergänzt, arbeitet das Gerät als Sauerstoff-Puls-Kardio-Respirograf. In der höchsten Ausbaustufe, bevorzugt für den Forschungseinsatz, sind auch EMG-Elektroden 50, Magensonde 70 und EEG-Elektroden angeschlossen. Ein modulartiger Aufbau des Gehäuses 1 und der Elektronikeinheit 4 ermöglicht dies.

Ferner ist ein Eingang 100 für einen Druck-Sensor zum Messen des Drucks im n-CPAP-Gerät vorhanden, mit dem die Effektivität der n-CPAP-Therapie gleichzeitig kontrolliert werden kann.

Die über 24 oder 48 Stunden kontinuierlich aufgezeichneten Meßwerte werden nach Rückkehr des Gerätes in der ärztlichen Praxis in den Computer überspielt und auf einem Display angezeigt bzw. über eine entwickelte Software ausgewertet und ausgedruckt. Die Feinauswertung aller aufgezeichneten Werte erfolgt ebenfalls mittels Computer, an den die Daten über eine Datenschnittstelle 6 übertragen werden. Damit kann dann auch die richtige Behandlung eingeleitet werden. Es können viele Patienten in kürzester Zeit nacheinander überwacht werden. Eine stationäre Einweisung der Patienten kann meist entfallen.

Je nach Ausbaustufe des Gerätes kann ein vollständiges Atembild des Patienten aufgezeichnet werden, einschließlich aller einschlägiger Körperfunktionen, so daß auch Begleiteffekte erkannt werden können, d. h. nicht nur Apnoen sondern auch Hypoventilationen, Herzrhythmusstörungen wie Extrasystolen oder Tachykardien, Muskelzittern, pH-Wert-Veränderungen usw. Mit Hilfe der EEG-Messung können parallel dazu die Wach- und die verschiedenen Schlafphasen erfaßt werden. Es ist auch möglich, zu Beginn der Messung typische Körperreaktionen des Patienten aufzuzeichnen, z. B. Luftanhalten, Luftpressen (Valsalva- bzw. Müller-Manöver), Husten, Tiefatmung etc., und diese unter Umständen sogar in die Elektronik einzueichen.

Wird der Thermosensor (Atemfühler) durch ein Volumen-Meßgerät ersetzt und/oder ergänzt, ist das oben genannte Gerät auch als portables Lungenfunktionsgerät einsetzbar und z. B. zur überwachung einer antiobstruktiven Asthma-Therapie geeignet.

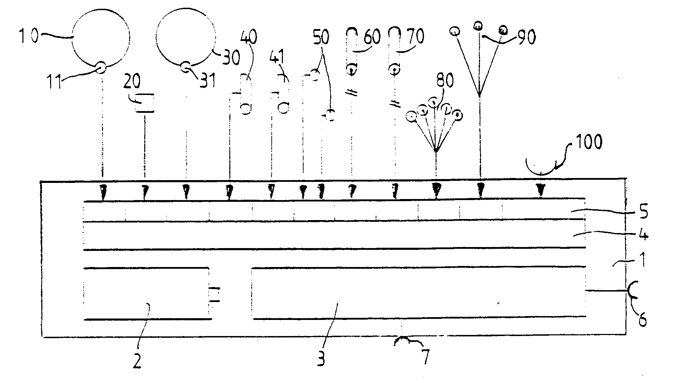

## Patentansprüche

1. Sauerstoff-Puls-Respirograf für die ambulante aber auch stationäre Anwendung, enthaltend:
- ein tragbares Gehäuse (1),
- eine Stromquelle (2) im Gehäuse (1),
- einen Langzeit-Signalspeicher (3) im Gehäuse (1),
- eine Elektronik-Einheit (4) im Gehäuse (1),
- einen Sensor (20), der auf die Atmung anspricht,
- einen Pulssensor (40),
- einen Blutsauerstoff-Sättigungssensor (41),
- eine Anpassungsschaltung (5) für die Sensoren in der Elektronikeinheit (4),
gekennzeichnet durch:
- einen Brustgurt (10) und/oder einen Bauchgurt (30) mit Dehnungssensor (11, 31),
- pH-Wert-Sensoren (60) zur Aufnahme von Meßwerten aus Speiseröhre und/oder Magen, und
- Auslegung von Stromquelle (2) und Langzeit-Signalspeicher (3) für einen 24- bis 48-stündigen Dauerbetrieb.

2. Sauerstoff-Puls-Respirograf nach Anspruch 1, gekennzeichnet durch
- einen Kombinationssensor auf der Grundlage der Lichttransmissionsmessung zur Messung von Puls und Blutsauerstoffsättigung.

3. Sauerstoff-Puls-Respirograf nach Anspruch 1 oder 2, gekennzeichnet durch
- Ausbildung des Sensors (20) als Thermofühler, der auf den Atemhauch anspricht.

4. Sauerstoff-Puls-Respirograf nach einem der Ansprüche 1 bis 3, gekennzeichnet durch
- Ausbildung des Sensors (20) als Windfahne, deren Stellung bzw. Bewegung elektrisch, magnetisch bzw. optisch abgetastet wird.

5. Sauerstoff-Puls-Respirograf nach einem der Ansprüche 1 bis 4, gekennzeichnet durch
- Klebe-Elektroden (50, 80, 90) zur Aufnahme eines Elektromyogramms, eines Elektrokardiogramms bzw. eines Elektroencephalogramms.

6. Sauerstoff-Puls-Respirograf nach einem der Ansprüche 1 bis 5, gekennzeichnet durch
- Drucksensoren (70) zur Aufnahme von Meßwerten aus der Speiseröhre bzw. dem Nasen-, Mund- und Rachenraum.

7. Sauerstoff-Puls-Respirograf nach einem der Ansprüche 1 bis 6, gekennzeichnet durch
- ein Magnetband bzw. eine Magnetplatte als Langzeit-Signalspeicher (3).

8. Sauerstoff-Puls-Respirograf nach einem der Ansprüche 1 bis 6, gekennzeichnet durch
- einen Halbleiterspeicher, insbesondere in Form einer Flash-Card, als Langzeit-Signalspeicher (3).

9. Sauerstoff-Puls-Respirograf nach einem der Ansprüche 1 bis 8, gekennzeichnet durch
- eine Datenschnittstelle (6) für die Verbindung zu einem die aufgezeichneten Werte analysierenden Computer.

10. Sauerstoff-Puls-Respirograf nach einem der Ansprüche 1 bis 9, gekennzeichnet durch
- modulartigen Aufbau des Gerätes (1) und/oder der Elektronikeinheit (4).

## Claims

1. An oxygen-pulse respirograph for ambulatory or stationary use, comprising:
- a portable housing (1),
- a power source (2) in the housing (1),
- a long-term signal store (3) in the housing (1),
- an electronic unit (4) in the housing (1),
- a sensor (20) which responds to breathing,
- a pulse sensor (40),
- a blood-oxygen saturation sensor (41),
- an adapter circuit (5) for the sensors in the electronic unit (4),
characterised by:
- a chest strap (10) and/or an abdominal strap (30) with an expansion sensor (11, 31),
- ph-value sensors (60) for recording measured values from the oesophagus and/or stomach, and
- design of the power source (2) and the long-term signal store (3) for 24 to 48 hour continuous operation.

2. An oxygen-pulse respirograph according to Claim 1, characterised by
- a combination sensor based on light-transmission measurement for measuring pulse and blood-oxygen saturation.

3. An oxygen-pulse respirograph according to Claim 1 or 2, characterised by
- designing the sensor (20) as a heat sensor which responds to breath.

4. An oxygen-pulse respirograph according to any one of Claims 1 to 3, characterised by
- designing the sensor (20) as a wind vane, the position and/or movement of which is sensed electrically, magnetically and/or optically.

5. An oxygen-pulse respirograph according to any one of Claims 1 to 4, characterised by
- adhesive electrodes (50, 80, 90) for recording an electromyogram, an electrocardiogram and/or an electroencephalogram.

6. An oxygen-pulse respirograph according to any one of Claims 1 to 5, characterised by
- pressure sensors (70) for recording measured values from the oesophagus and/or nose cavity, mouth cavity and pharynx.

7. An oxygen-pulse respirograph according to any one of Claims 1 to 6, characterised by
- a magnetic tape or a magnetic disc as long-term signal store (3).

8. An oxygen-pulse respirograph according to any one of Claims 1 to 6, characterised by
- a semiconductor memory, in particular in the form of a flash card, as long-term signal store (3).

9. An oxygen-pulse respirograph according to any one of Claims 1 to 8, characterised by
- a data interface (6) for the connection to a computer analysing the recorded values.

10. An oxygen-pulse respirograph according to any one of Claims 1 to 9, characterised by
- modular construction of the apparatus (1) and/or of the electronic unit (4).

## Revendications

1. Respirographe oxygène-pouls pour application ambulante mais également stationnaire, contenant :
- un boîtier (1) portatif,
- une source électrique (2) se trouvant dans le boîtier (1),
- une mémoire de signaux (3) à stockage de longue durée, dans le boîtier (1),
- une unité électronique (4) dans le boîtier (1),
- un capteur (20) réagissant à la respiration,
- un capteur de pouls (40),
- un capteur de saturation du sang en oxygène (41),
- un circuit d'adaptation (5) pour les capteurs, dans l'unité électronique (4),
caractérisé par :
- une ceinture thoracique (10) et/ou une ceinture abdominale (30) avec un capteur extensométrique (11, 31),
- des capteurs de pH (60) pour capter les valeurs de mesure issues de l'oesophage et/ou de l'estomac, et
- la source électrique (2) et la mémoire à signaux (3) à stockage de longue durée étant conçues pour un fonctionnement permanent, pendant une durée de 24 à 48 heures.

2. Respirographe oxygène-pouls, selon la revendication 1, caractérisé par
- un capteur de combinaison, basé sur la mesure de la transmission de la lumière, pour mesurer le pouls et la saturation du sang en oxygène.

3. Respirographe oxygène-pouls, selon la revendication 1 ou 2, caractérisé par
- la réalisation du capteur (20) sous forme de thermosonde, réagissant au souffle respiratoire.

4. Respirographe oxygène-pouls, selon l'une quelconque des revendications 1 à 3, caractérisé par
- la réalisation du capteur (20) sous forme de drapeau soumis à l'effet du vent, dont la position respectivement le déplacement est exploré par voie électrique, magnétique ou optique.

5. Respirographe oxygène-pouls, selon l'une quelconque des revendications 1 à 4, caractérisé par
- des électrodes adhésives (50, 80, 90), destinées à l'enregistrement d'un électromyogramme, d'un électrocardiogramme, respectivement d'un électroencéphalogramme.

6. Respirographe oxygène-pouls, selon l'une quelconque des revendications 1 à 5, caractérisé par
- des capteurs de pression (70) pour enregistrer des valeurs de mesure issues de l'oesophage, respectivement de la cavité nasale, buccale et du pharynx.

7. Respirographe oxygène-pouls, selon l'une quelconque des revendications 1 à 6, caractérisé par
- une bande magnétique, respectivement un disque magnétique, faisant office de mémoire de signaux (3) stockant sur une longue durée.

8. Respirographe oxygène-pouls, selon l'une quelconque des revendications 1 à 6, caractérisé par
- une mémoire à semi-conducteur, en particulier sous la forme d'une carte flash, faisant office de mémoire à signaux (3) à stockage de longue durée.

9. Respirographe oxygène-pouls, selon l'une quelconque des revendications 1 à 8, caractérisé par
- une interface de données (6), pour la liaison à un ordinateur effectuant l'analyse des valeurs enregistrées.

10. Respirographe oxygène-pouls, selon l'une quelconque des revendications 1 à 9, caractérisé par
- une structure modulaire de l'appareil (1) et/ou de l'unité électronique (4).
